# EUROPEAN PATENT APPLICATION

(11) **EP 3 626 280 A1**
(43) Date of publication of application: **25.03.2020**
(21) Application number: 19197968.1
(22) Date of filing: 18.09.2019
(51) Int. Cl.: A61M 1/16

(54) **PRECISION RECHARGING BASED ON SORBENT EFFLUENT ANAYSIS**

(30) Priority: 19.09.2018 US 201862733229 P; 10.06.2019 US 201916436006
(71) Applicant: Medtronic, Inc., Minneapolis, MN 55432 (US)
(72) Inventor: PUDIL, Bryant J., Plymouth, MN Minnesota 55447 (US); HOBOT, Christopher M., Rogers, MN Minnesota 55374 (US); GERBER, Martin T, Maple Grove, MN Minnesota 55369 (US)
(74) Representative: Maschio, Antonio

(57) **Abstract**

The invention relates to devices, systems, and methods for precision recharging of sorbent materials in a sorbent module. The devices, systems, and methods use sensor-based analysis of an effluent of the sorbent module during recharging to set recharge parameters used in recharging the sorbent material.

## Description

### CROSS-REFERENCE TO RELATED APPLICATION

This application claims the benefit of and priority to U.S. Provisional Patent Application No. 62/733,229 filed September 19, 2018, the entire disclosure of which is incorporated by reference herein.

### FIELD OF THE INVENTION

The invention relates to devices, systems, and methods for precision recharging of sorbent materials in a sorbent module. The devices, systems, and methods use sensor-based analysis of an effluent of the sorbent module during recharging to set recharge parameters used in recharging the sorbent material.

### BACKGROUND

Zirconium phosphate and zirconium oxide are common sorbent materials used in sorbent dialysis. The zirconium phosphate removes cations, such as potassium, calcium, magnesium, and ammonium ions from spent dialysate. Zirconium oxide removes anions, such as phosphate or fluoride anions from spent dialysate. After use, the zirconium phosphate and zirconium oxide can be reprocessed or recharged to restore a functional capacity or a range of functional capacity of the sorbent materials.

Known recharging systems do not control the volume of recharge solutions used in recharging the zirconium phosphate and zirconium oxide, and instead simply treat the materials with enough recharging chemicals to ensure complete recharging assuming the sorbent materials have been fully saturated or depleted. Complete recharging of the sorbent materials is generally used to cover worst case situations to avoid ammonia breakthrough in patients with high levels of urea or other ions. Complete recharging of the sorbent materials in most cases is wasteful, more costly, and more time consuming, than recharging the sorbent materials needs to be, while still meeting necessary safety standards. The known sorbent material recharging systems do not take into account variability including effects from a particular type of dialysis being performed, subsequent recharging setups, types and quantities of materials and fluids used during recharging, and the specific conditions for a particular recharging setup. The known systems and methods do not allow technicians or users to accurately and effectively provide a cost effective recharge of a sorbent material. Rather, the known systems and methods rely on a one-size-fits-all approach to sorbent material recharging that relies on averages without any consideration of conditions, features, and factors specific to individuals, machines, treatment facilities, or any other variable having an impact on sorbent material recharging.

Hence, there is a need for precision sorbent material recharging systems and methods. The need extends to systems and methods that can use sensors in a recharging flow path to set a volume of recharge solutions used. The need generally includes sorbent material recharging that can monitor a sorbent material recharging process using sensors that account for variance in a specific recharging setup, the types and quantities of materials and fluids used for sorbent material recharging, and the specific machine and environmental conditions present during sorbent material recharging. The need extends to performing precision sorbent recharging accurately, efficiently, and economically. The need includes systems and methods that allow for such precision recharging without the need for additional information about a patient or the previous dialysis sessions.

### SUMMARY OF THE INVENTION

The first aspect of the invention relates to a system. In any embodiment, the system can comprise (a) a sorbent recharger, the sorbent recharger comprising a recharging flow path, the recharging flow path fluidly connectable to a sorbent module containing at least one sorbent material; (b) at least one recharge solution source containing at least one recharge solution for recharging the at least one sorbent material within the sorbent module, the at least one recharge solution source fluidly connectable to a sorbent module inlet; (c) at least one pump in the recharging flow path; (d) at least a first sensor in an effluent line fluidly connectable to a sorbent module outlet; and (e) a processor; the processor programmed to receive data from the first sensor and to set at least one recharge parameter for recharging of the at least one sorbent material.

In any embodiment, the at least one sorbent material can comprise zirconium phosphate.

In any embodiment, the at least one sorbent material can comprise zirconium oxide.

In any embodiment, the at least one recharge parameter can be selected from the group consisting of a volume of at least one recharge solution introduced through the sorbent module, a concentration of at least one recharge solution introduced through the sorbent module, and a flow rate of at least one recharge solution introduced through the sorbent module.

In any embodiment, the system can comprise at least a second sensor positioned between the at least one recharge solution source and the sorbent module inlet.

In any embodiment, the processor can be programmed to set the at least one recharge parameter based on a difference between data received from the first sensor and data received from the second sensor.

In any embodiment, the processor can be programmed to introduce the at least one recharge solution into the sorbent module until a pH or conductivity in an effluent of the sorbent module is within a predetermined range of a pH or conductivity of the at least one recharge solution.

In any embodiment, the first sensor can be a pH sensor or a conductivity sensor.

In any embodiment, the first sensor can be an ion selective electrode.

In any embodiment, the first sensor can be an optical sensor.

In any embodiment, the processor can be programmed to compare a conductivity or pH in an effluent of the sorbent module to an elution profile.

In any embodiment, the sorbent module can contain zirconium phosphate, and the at least one recharge solution source can comprise at least a water source and a brine source.

In any embodiment, the sorbent module can contain zirconium oxide, and the at least one recharge solution source can comprise at least a base source.

In any embodiment, the system can perform precision recharging of the at least one sorbent material.

The features disclosed as being part of the first aspect of the invention can be in the first aspect of the invention, either alone or in combination, or follow a preferred arrangement of one or more of the described elements.

The second aspect of the invention is drawn to a method. In any embodiment, the method can comprise the steps of introducing a recharge solution into a sorbent module containing at least one sorbent material in a sorbent recharger; receiving data from a first sensor positioned in an effluent line fluidly connected to a sorbent module outlet; and setting at least one recharge parameter based on data received from the first sensor.

In any embodiment, the sorbent module can contain zirconium phosphate.

In any embodiment, the sorbent module can contain zirconium oxide.

In any embodiment, the at least one recharge parameter can be selected from the group consisting of a volume of at least one recharge solution introduced through the sorbent module, a concentration of at least one recharge solution introduced through the sorbent module, and a flow rate of at least one recharge solution introduced through the sorbent module.

In any embodiment, the method can comprise the step of receiving data from a second sensor positioned between a recharge solution source containing the recharge solution and a sorbent module inlet; and comparing the data from the second sensor to the data from the first sensor.

In any embodiment, the method can comprise the step of stopping introduction of the recharge solution when data received from the first sensor is within a predetermined range of data received from the second sensor.

In any embodiment, the first sensor can be a pH sensor, a conductivity sensor, an ion selective electrode, or an optical sensor.

In any embodiment, the recharge solution can comprise at least a brine solution.

In any embodiment, the recharge solution can comprise at least a base solution.

In any embodiment, the step of setting the at least one recharge parameter can comprise comparing data from the first sensor to an elution profile of the sorbent module.

In any embodiment, the first sensor can be an ion selective electrode, and the method can comprise the step of stopping introduction of the recharge solution when a concentration of at least one ion in the effluent line is within a predetermined range.

In any embodiment, the predetermined range can be a non-zero value.

In any embodiment, the method can be performed by the system of the first aspect of the invention.

In any embodiment, the at least one recharge parameter can be set based on data received from the first sensor at a first time, and the at least one recharge parameter can be adjusted based on data received from the first sensor at a second time.

In any embodiment, the method can be for precision recharging of the at least one sorbent material.

The features disclosed as being part of the second aspect of the invention can be in the second aspect of the invention, either alone or in combination, or follow a preferred arrangement of one or more of the described elements.

### BRIEF DESCRIPTION OF THE DRAWINGS

FIG. 1 shows a sorbent recharger for recharging a sorbent material in a sorbent module.
FIG. 2 shows a sorbent recharger connected to recharge solution sources for recharging sorbent materials.
FIG. 3 shows a zirconium phosphate recharging flow path.
FIG. 4 shows a zirconium oxide recharging flow path.
FIG. 5 is a flow chart showing a method for precision recharging of a sorbent material based on sorbent effluent analysis.

### DETAILED DESCRIPTION OF THE INVENTION

Unless defined otherwise, all technical and scientific terms used have the same meaning as commonly understood by one of ordinary skill in the art.

The articles "a" and "an" are used to refer to one or to over one (*i*.*e*., to at least one) of the grammatical object of the article. For example, "an element" means one element or over one element.

The term "adjusted," or to "adjust," refers to changing one or more parameters in a process or method.

The phrase "based on" can refer to using information or data obtained by any means wherein the use can be of any form including performing calculations of determined or observed parameters, determining values, transmitting determined or observed values, measuring values, or processing the obtained information or data in any fashion known to those of skill in the art. For example, the phrase "based on data" can refer to performing a calculation or determining one or more value or variable using data.

A "base solution" can be a solution having a pH greater than 7. In certain embodiments, the base solution can contain either a Lewis base or a Bronsted-Lowry base.

A "base source" is a fluid or concentrate source from which a base solution can be obtained.

A "brine solution" can be a solution containing salts and/or buffers containing solutes used in recharging a sorbent material. In certain embodiments, the brine solution can be a solution of a sodium salt, acetic acid, sodium acetate, or combinations thereof.

The term "brine source" refers to a source of a solution of salts and/or buffers containing solutes used in recharging a sorbent material. In certain embodiments, the brine source can contain a sodium salt, acetic acid, sodium acetate, or combinations thereof.

The terms "contain," "to contain," and "containing" when used in reference to a material refers to retaining that material as contents of a compartment, module, device, or structure.

The term "compare," "comparing," and the like refers to the process of determining the similarity or difference between two values, states, parameters, or data.

The term "comprising" includes, but is not limited to, whatever follows the word "comprising." Use of the term indicates the listed elements are required or mandatory but that other elements are optional and may be present.

The term "concentration" refers to an amount of a solute dissolved in a given unit of solvent.

The term "conductivity" refers to a measurement of the ability for electrons to move through a fluid or substance. The conductivity can be the inverse of the electrical resistance of the fluid or substance.

The term "conductivity sensor" refers to a device for measuring conductance, or the inverse of the electrical resistance, of a fluid or substance.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The term "difference," when referring to data from two different sources, refers to a deviation of data from a first source from that of a second source.

The term "effluent" can refer to liquid, gas, or a combination thereof exiting a container, compartment, or cartridge.

An "effluent line" can be a fluid passageway, tube, or path of any kind into which liquid, gas, or a combination thereof exiting a container, module, or component can flow.

An "elution profile" refers to values for fluid parameters of an effluent of a sorbent module as a function of an initial state of the sorbent module.

The term "fluidly connectable" refers to a capability for providing for the passage of fluid, gas, or combination thereof, from one point to another point. The capability of providing such passage can be any connection, fastening, or forming between two points to permit the flow of fluid, gas, or combinations thereof. The two points can be within or between any one or more of compartments of any type, modules, systems, components, such as rechargers, as described herein.

The term "fluidly connected" refers to a particular state such that the passage of fluid, gas, or combination thereof, is provided from one point to another point. The connection state can also include an unconnected state, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

A "free chlorine solution" refers to a solution having solutes that can generate chlorine, hypochlorite ions, or hypochlorous acid either as a gas or in solution.

A "free chlorine source" refers to a source of a substance that can generate chlorine, hypochlorite ions, or hypochlorous acid either as a gas or in solution.

The terms "introducing," "introduced," or to "introduce" refers to directionally moving or flowing a fluid, a gas, or a combination thereof by any means known to those of skill in the art.

An "ion" is an atom or molecule having a charge.

An "ion selective electrode" is an electroanalytical sensor capable of detecting a concentration of a specified ion in solution.

The term "non-zero value" refers to any number that is not zero. In certain embodiments, the non-zero value can be any number greater than zero.

An "optical sensor" refers to a sensor that can convert light rays into electronic signals. The optical sensor measures the physical quantity of light, which can be at specific wavelengths, and then translates the measurements into a form that is readable by an instrument. An optical sensor can be part of a larger system that integrates a source of light, a measuring device and the optical sensor.

The term "pH" refers to the negative log of the hydrogen ion concentration in a solution.

The term "pH sensor" refers to a device for measuring the pH or hydrogen ion concentration of a fluid.

The term "positioned" refers to a component connected to or in contact with the feature being referred to. The contact can be fluid or electrical and is intended to be used in the broadest reasonable interpretation.

"Precision recharging" refers to recharging a sorbent material to a desired state without the need for excess chemicals or solutions. The precision recharging can include using input data or parameters to determine the precise need or amount of chemicals or solutions and length of time required.

The term "predetermined range" can be any range of possible values for a parameter obtained in advance or a priori to actual use in a method.

The term "processor" as used is a broad term and is to be given its ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. In any embodiment of the first, second, third, and fourth invention, the terms can include ROM ("read-only memory") and/or RAM ("random-access memory") associated therewith.

The term "programmed," when referring to a processor, can mean a series of instructions that cause a processor to perform certain steps. For example, a processor can be "programmed" to set functions, parameters, variables, or instructions.

The term "pump" refers to any device that causes the movement of fluids or gases by applying suction or pressure.

The terms "receiving," "to receive," or "received" in the context of data refers to obtaining information or any other means of data transmission or representation from any source by any means including direct electrical contact, induction, magnetic, wireless transmission, or networked connection.

A "recharge parameter" is any factor or variable used in recharging. In certain embodiments, a recharge parameter can include one or more of a flow rate, concentration, or volume of recharge solutions used in recharging.

A "recharge solution" or "recharge solutions" can be a solution containing appropriate ions for recharging a specific sorbent material. A recharge solution can be a single solution containing all necessary ions for recharging a sorbent material. Alternatively, the recharge solution can contain some of the ions for recharging the sorbent material, and one or more other recharge solutions can be used to form a composite "recharge solution" to recharge the sorbent material, as described herein.

A "recharge solution source" can be any fluid or concentrate source from which a recharge solution can be stored, obtained, or delivered therefrom.

"Recharging" refers to treating a sorbent material to restore a functional capacity of the sorbent material putting the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged."

A "recharging flow path" can be a path through which fluid can travel while recharging a sorbent material in a sorbent module.

The term "sensor," as used herein, can be a converter of any type that can measure a physical property or quantity of a matter in a solution, liquid or gas, and can convert the measurement into a signal which can be read by an electronic instrument. A sensor may include either a conductivity sensor, a pH sensor, or an optical sensor.

The term "setting," "set," or "to set" in the context of performing a series of instructions or steps refers to the process of adjusting or controlling one or more variable to a desired value for use in a process, method, or system.

A "sorbent cartridge module" or "sorbent module" means a discreet component of a sorbent cartridge. Multiple sorbent cartridge modules can be fitted together to form a sorbent cartridge of two, three, or more sorbent cartridge modules. The "sorbent cartridge module" or "sorbent module" can contain any selected materials for use in sorbent dialysis and may or may not contain a "sorbent material" or adsorbent, and less than a full complement of one or more sorbent material needed for performing a sorbent function. In other words, the "sorbent cartridge module" or "sorbent module" generally refers to the use of the "sorbent cartridge module" or "sorbent module" in sorbent-based dialysis, e.g., REDY (REcirculating DYalysis), and not that a "sorbent material" that is necessarily contained in the "sorbent cartridge module" or "sorbent module."

"Sorbent materials" are materials capable of removing specific solutes from solution, such as cations or anions.

The term "sorbent module inlet" can refer to a portion of a sorbent module through which fluid, gas, or a combination thereof can be drawn into the sorbent module.

The term "sorbent module outlet" can refer to a portion of a sorbent module through which fluid, gas, or a combination thereof can flow or be drawn out of the sorbent module.

A "sorbent recharger" or "recharger" is an apparatus designed to recharge at least one sorbent material, and optionally one or more non-sorbent material.

The term "stopping" or to "stop" refers to ceasing or causing to be ceased a process or function.

The term "volume" refers to the three dimensional space occupied by a substance or container.

A "water source" can be a fluid source from which water can be stored, obtained, or delivered therefrom.

"Zirconium oxide" is a sorbent material that removes anions from a fluid, exchanging the removed anions for different anions. Zirconium oxide can also be formed as hydrous zirconium oxide.

"Zirconium phosphate" is a sorbent material that removes cations from a fluid, exchanging the removed cations for different cations.

### Precision Recharging

The invention is drawn to systems and methods that can provide for precision recharging of a sorbent material within a sorbent module that is not limited to any particular standard but can be dependent upon input variables to achieve a desired state. Precision recharging is an approach for recharging a sorbent material that takes into account individual variability of the sorbent cartridge, state of materials, and conditions being used to recharge the sorbent material. The precision recharging can reflect, but necessarily include, the implied effects from a particular dialysis and recharging setup, types and quantities of materials and fluids used for sorbent material recharging, and specific conditions for a particular recharging setup. Precision recharging can provide for more accurate, effective, and/or economical recharging of a sorbent material. The approach is contrasted to a one-size-fits-all approach, in which sorbent material recharging relies on averages without any consideration of specific conditions, features, and factors, or any other variable that might have an impact on sorbent recharging.

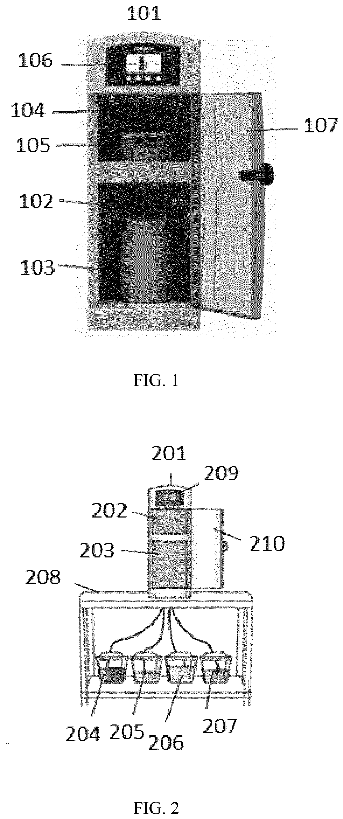

FIG. 1 illustrates a non-limiting embodiment of a sorbent recharger **101** for recharging zirconium phosphate in a zirconium phosphate sorbent module **103** and/or zirconium oxide in a zirconium oxide sorbent module **105.** The sorbent recharger **101** can include a receiving compartment **102** for receiving a zirconium phosphate sorbent module **103.** Fluid connections (not shown in FIG. 1) connect to the top and bottom of the zirconium phosphate sorbent module **103** for introducing recharge solutions into, through and out of the zirconium phosphate sorbent module **103.** The recharge solutions replace ions bound to the sorbent materials during dialysis with new ions, recharging the zirconium phosphate within the zirconium phosphate sorbent module **103** and/or zirconium oxide in a zirconium oxide sorbent module **105**, allowing reuse of the sorbent modules **103** and **105** in dialysis. The sorbent recharger **101** can include a second receiving compartment **104** for receiving a second sorbent module, such as zirconium oxide sorbent module **105**, which is also fluidly connected to recharge solution sources for recharging of zirconium oxide sorbent module **105.** The sorbent recharger **101** can include any number of receiving compartments for receiving any number of zirconium phosphate and/or zirconium oxide sorbent modules. In certain embodiments, a sorbent recharger can recharge only a single sorbent material, such as a sorbent recharger with one or more receiving compartments each for receiving sorbent modules containing the same sorbent materials. Alternatively, the sorbent recharger **101** can include multiple receiving compartments for receiving sorbent modules containing different sorbent materials, as illustrated in FIG. 1. A user interface **106** can be provided to start or control the recharging process by the user and to receive information from the sorbent recharger **101**, such as the volume of one or more recharge solutions necessary for recharging. The user interface **106** can also provide the status of the recharging process to the user, such as the time to completion for each recharging step, or a time to complete the entire recharging process. User interface **106** provides alert messages if any problems are detected during recharging, such as leaks, occlusions, pump failures, or mismatched chemicals. A door **107** on the sorbent recharger **101** controls access to the receiving compartments **102** and **104** during operation.

The sorbent recharger **101** can have one or more processors for receiving data from one or more sensors and setting one or more recharge parameters, such as a volume of recharge solution, a concentration of recharge solution, or and/or a flow rate of recharge solution necessary for recharging the sorbent modules. The sorbent recharger **101** can transmit data obtained from a sensor wirelessly or by wired connection. The sorbent recharger **101** can be connected to a local area network (LAN) or a secure internet connection that transmits the required instructions or data to a component that either process the data or performs a set of instructions. It will be understood that the determination and setting of the recharge parameters is not limited to a component physically attached to the recharger or other component local to the dialysis system, but can be performed at any local or remote data center including cloud infrastructure, or other network of remote servers hosted on the Internet that can store, manage, and process data. The networked systems can be secured by any known methods and procedures as known to those of skill in the art. Although illustrated in FIG. 1 as having two receiving compartments **102** and **104,** a sorbent recharger for recharging a single sorbent material can have a single receiving compartment or multiple receiving compartments for receiving and recharging multiple modules containing the same sorbent material. Sorbent rechargers with any number of receiving compartments for recharging any number or combination of zirconium oxide and/or zirconium phosphate sorbent modules can be constructed. For example, a sorbent recharger with two zirconium phosphate receiving compartments and two zirconium oxide receiving compartments can be similarly constructed. The rechargers can have 1, 2, 3, 4, 5, 6, or more receiving compartments, each capable of receiving zirconium oxide or zirconium phosphate sorbent modules.

FIG. 2 illustrates a non-limiting embodiment of a sorbent recharger **201** set up for recharging zirconium phosphate and/or zirconium oxide. To recharge the zirconium phosphate and/or zirconium oxide, one or more recharge solutions can be passed through a zirconium phosphate sorbent module. As shown in FIG. 2, the sorbent recharger **201** can be fluidly connected to one or more recharge solution sources, such as water source **204**, brine source **205**, base source **206**, and disinfectant source **207.** The brine source **205** can contain a brine solution having a sodium salt and buffer. As an alternative, separate sodium and buffer sources can be used, or the buffer can be replaced by separate acid and base sources. Any number of recharge solution sources can be included. The sorbent recharger **201** has a zirconium phosphate receiving compartment **203** and/or a zirconium oxide receiving compartment **202.** As described, the sorbent recharger **201** can include any number of receiving compartments for receiving any combination of zirconium oxide and zirconium phosphate sorbent modules. The sorbent recharger **201** can also include one or more pumps and valves (not shown in FIG. 2) for selectively introducing the recharge solutions from the recharge solution sources to the sorbent modules. As shown in FIG. 2, the recharge solution sources are housed external to the sorbent recharger **201.** Alternatively, the recharge solution sources can be housed within the sorbent recharger **201.** A drain line (not shown) can be connected to the sorbent recharger **201** for disposal of waste fluids exiting the sorbent modules. The drain line can be fluidly connected to a drain, or alternatively, the drain line can be fluidly connected to one or more waste reservoirs for storage and later disposal. As illustrated in FIG. 2, the sorbent recharger **201** can be small enough to fit on top of a table **208.** However, larger sorbent rechargers can be used. A user interface **209** can allow user control of the recharging process and provide messages concerning the recharging. Door **210** controls access to the receiving compartments **202** and **203** during recharging.

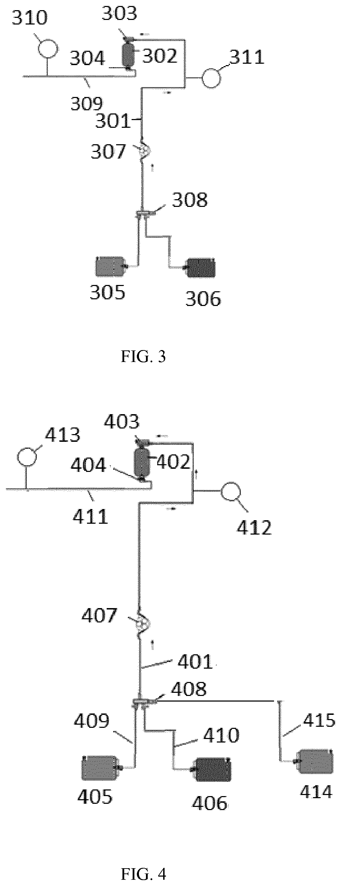

The sorbent rechargers can include one or more recharging flow paths fluidly connected to the sorbent modules. FIG. 3 illustrates a non-limiting embodiment of a zirconium phosphate recharging flow path **301** for recharging zirconium phosphate in a zirconium phosphate sorbent module **302.** After dialysis, the zirconium phosphate sorbent module **302** can be removed from the dialysis system and placed in the recharger. The zirconium phosphate sorbent module **302** can be fluidly connectable to the zirconium phosphate recharging flow path **301** through zirconium phosphate sorbent module inlet **303** and zirconium phosphate sorbent module outlet **304.** The zirconium phosphate recharging flow path **301** can include at least one pump **307** to provide a driving force for moving fluids through the zirconium phosphate recharging flow path **301.** In certain embodiments, two or more pumps can be included for individually introducing fluids from the recharge solution sources into the zirconium phosphate sorbent module **302.** As described, the system and methods allow for setting a flow rate of the recharge solutions through the zirconium phosphate recharging flow path **301** for precision recharging. The zirconium phosphate recharging flow path **301** can include one or more recharge solution sources, including a brine source **305** and a water source **306.** The brine source **305** can contain a brine solution of a salt, such as sodium chloride, and a buffer, such as a mixture of sodium acetate and acetic acid. The sodium and hydrogen ions in the recharge solution displace the ammonium ions and other cations adsorbed by the zirconium phosphate during a prior dialysis session. Although shown as a single brine source **305**, multiple recharge solution sources can be used. For example, a first recharge solution source containing sodium chloride and a second recharge solution source containing an acetate buffer. Alternatively, three recharge solution sources can be used, with sodium chloride, sodium acetate, and acetic acid in separate recharge solution sources. If multiple recharge solution sources are used, the recharge solutions can be mixed within the zirconium phosphate recharging flow path **301** or pumped through the zirconium phosphate sorbent module **302** sequentially. Any combination of sodium salt and buffer capable of causing exchange of ammonium, potassium, calcium, and magnesium for sodium and hydrogen ions can be used as the recharge solutions. The water from water source **306** can be used to dilute the brine solution from brine source **305** if a concentrated brine solution is used, and to rinse the zirconium phosphate sorbent module **302** before and after introducing the brine solution through the zirconium phosphate sorbent module **302.** In certain embodiments, the brine source **305** can contain a brine concentrate having a concentration of salt and buffer greater than that to be used in recharging. The brine solution can be diluted in-line with water from the water source **306** to generate a recharge solution with a desired concentration, as described. Optional valve **308** can be included to control the movement of fluid from either the brine source **305** or water source **306.** Alternatively, separate pumps on fluid lines fluidly connected to each recharge solution source can be used. A processor (not shown) can be programmed to control the pumps or valves to direct recharge solutions from the recharge solution sources through the zirconium phosphate sorbent module **302.** One of skill in the art will understand that multiple pump and valve arrangements can be used to pump the necessary recharge solutions through the zirconium phosphate sorbent module **302.**

A sensor **310** positioned in, or in fluid connection, with the effluent line **309** can determine at least one fluid parameter of the effluent recharge solution in effluent line **309.** In certain embodiments, the sensor **310** can be an ion selective electrode, a pH sensor, a conductivity sensor, an ion selective electrode, an optical sensor, or a combination thereof. As described, data from the sensor **310** in, or in fluid connection, with effluent line **309** can be received by the processor to set one or more recharge parameters, such as a volume of the recharge solutions necessary for recharging the zirconium phosphate, a flow rate of the recharge solutions to be used in recharging, or a concentration of the recharge solutions.

During a dialysis session, the zirconium phosphate can remove cations from spent dialysate, including ammonium, potassium, calcium, and magnesium, exchanging the cations for hydrogen and sodium ions. The ammonia is formed by the breakdown of urea by urease in the sorbent cartridge during treatment. The sodium chloride and buffer solutions used in recharging the zirconium phosphate serve to displace the cations absorbed during treatment with sodium and hydrogen ions, facilitating reuse of the zirconium phosphate.

The conductivity or pH of the effluent of the zirconium phosphate sorbent module **302** can be used to set the recharge parameters for recharging the zirconium phosphate. The conductivity and pH of eth effluent is a function of the ions in solution. If fully saturated with ammonium, calcium, magnesium, and potassium, the conductivity can have one value. As the recharging process progresses and the ammonium, calcium, magnesium, and potassium ions are displaced for sodium and hydrogen ions, the conductivity and pH can shift to approach that of the brine solution used. A graph of conductivity and/or pH versus recharge brine volume during recharging is an elution profile can be characterized. In certain embodiments, the conductivity or pH of the effluent at a given point in the recharging process can be compared to a known elution profile. Using a lookup table, the processor can compare the conductivity or pH to a known elution profile and determine the remaining capacity of the zirconium phosphate. The elution profile may need to be characterized for various combinations of ammonium, calcium, magnesium, and potassium ions and stored in a lookup table. When recharging starts, the processor can detect the conductivity and/or pH and compare the values to the lookup table to estimate the remaining capacity of the zirconium phosphate. The system can also calculate how much recharge brine volume is required to partially or fully recharge the zirconium phosphate capacity by using the elution profile. The system can then deliver the volume of recharge brine to achieve the desired level of recharge based on a concentration of the recharge solution and/or a flow rate of the recharge solution. Alternatively, the processor can set the flow rate or concentration of the recharge solutions based on data from the sensor using a set volume of recharge solution to be used. The processor can receive the conductivity or pH of the effluent from the zirconium phosphate sorbent module **302** at the beginning of the recharging process, after the recharging process has started, or at one or more times during recharging. One of skill in the art will understand that there are interdependencies between concentration, time, volume and flow rate. For example, at a faster flow rate or at a higher recharge solution concentration, recharging the sorbent material may be quicker, but may require additional chemicals. At lower flow rates or recharge solution concentrations the process can be more efficient with respect to chemical usage, but can take longer. A user can input the values that are desired to be minimized based on the user objectives, and the system can determine the proper concentration, flow rate, and/or volume of the recharge solutions to minimize the desired parameters. The system can be adaptive to the needs of the user, with the user defining the desired objectives for the recharging process, such as to minimize time or to minimize chemical usage In certain embodiments, the recharger can use a higher concentration or flow rate at the beginning of recharging, and adjust the concentration and/or flow rate lower as the recharging process nears completion, as determined by the sensors, minimizing both the time and volume of recharge solutions necessary for recharging the zirconium phosphate.

In certain embodiments, the sensor **310** can be an ion selective electrode. An ion selective electrode can be used to determine the concentration of a specific ion in a fluid. The ion selective electrode can be selective for any ion that is adsorbed by the zirconium phosphate during dialysis, such as ammonium, calcium, potassium, or magnesium ions. As recharging progresses, the concentration of the ions adsorbed by the zirconium phosphate during treatment will decrease. When the concentration of the ions in the effluent approaches zero, the zirconium phosphate is at or near full capacity and the recharging process is ended. The processor can stop the introduction of the recharge solution when the concentration of any of the ions in the effluent line **309** is within a predetermined range. The predetermined range can be set based on a desired final recharge state. For example, the predetermined range can be set at or near 0 if full recharging is required or desired. Alternatively, the predetermined range can be set at some non-zero value if a full restoration of capacity is not needed or wanted. For example, for an incident (newly diagnosed) patient or an acute kidney injury patient with very high pre-dialysis BUN, full capacity of the zirconium phosphate may not be desired because treatment with a full capacity zirconium phosphate may lower the patient BUN too much in one session, causing disequilibrium syndrome due to excess removal of uremic solutes. The zirconium phosphate capacity can be limited to control the amount of urea that is removed during treatment. Alternatively, the predetermined range can be set at some non-zero value because the void volume of the sorbent cartridge will allow additional recharge solution to go through the sorbent cartridge during the next step of the recharge process. For example, the processor can determine that the next step in the recharge process, such as water rinsing, can be started when a specified non-zero value or concentration is measured at the outlet of the sorbent cartridge because the void volume of the sorbent cartridge will still contain recharge solution that is yet to come out and contact the sensor. As the recharge solution continues to exit the sorbent cartridge during the rinse step, the concentration or measured value will continue to decrease to the predetermined value targeted to indicate the end of the recharge process.

In certain embodiments, the sensor **310** can be an optical sensor. During recharging, protein fragments may be eluted from the zirconium phosphate sorbent module **302.** The protein fragments can be detected using an optical sensor. For example, the absorbance of light at 230 nm can be used to detect the presence and concentration of proteins or protein fragments. As the recharging process nears competition, protein fragments will no longer be eluted from the zirconium phosphate sorbent module **302.** As the absorbance of light at 230 nm approaches a background threshold, the processor can stop introduction of the recharge solutions. In certain embodiments, the optical sensor can be a colorimetric sensor. For example, one or more ammonia sensing membranes can be contacted with the effluent, the ammonia sensing membranes changing color or any other optical parameter in response to the ammonia content in the effluent line **309.** The optical change in the ammonia sensing membrane can be detected by a photodetector to determine the ammonia or ammonium content of the effluent. As the recharging process nears competition, the ammonia and/or ammonium concentration in the effluent will approach 0.

In certain embodiments, a second sensor **311** can be included in, or in fluid connection, with the zirconium phosphate recharging flow path **301** at a position upstream of the zirconium phosphate sorbent module **302.** As the recharging process nears completion, the difference in any fluid parameters of the initial recharge solution and the effluent of the zirconium phosphate sorbent module **302** decreases, or becomes constant, because the zirconium phosphate approaches equilibrium with the recharge solutions. As such, the difference in conductivity or pH as measured by the first sensor **310** and second sensor **311** can be used to determine when recharging is complete. In certain embodiments, the processor can be programmed to stop the introduction of recharging fluids when the conductivity or pH of the effluent is within a predetermined range of the conductivity or pH of the initial recharge solutions. As described, the predetermined range may be when the effluent and recharge solution have identical or nearly identical conductivities or pH for a full recharge of the zirconium phosphate. Alternatively, the predetermined range can be set at other values if a full capacity zirconium phosphate sorbent module is not needed or desired. One of skill in the art will understand that, if the recharge solutions have a known conductivity or pH, the second sensor **311** is unnecessary.

In certain embodiments, after introducing brine solution through the zirconium phosphate sorbent module **302**, the zirconium phosphate sorbent module **302** can be rinsed with water to remove any remaining brine. The amount of water used during the final rinse can be set by the processor using the sensor **310**, such as a conductivity sensor. The conductivity of the effluent can be a function of the ions present in solution. As the rinsing nears completion, the conductivity of the effluent will approach that of pure water because the ions from the brine solution are washed out of the zirconium phosphate sorbent module **302.** The processor can introduce water through the zirconium phosphate sorbent module **302** until the conductivity reaches a predetermined range, such as 2-mS/cm or less.

FIG. 4 illustrates a non-limiting embodiment of a zirconium oxide recharging flow path **401** for recharging zirconium oxide in a zirconium oxide sorbent module **402.** After dialysis, the zirconium oxide sorbent module **402** can be removed from the dialysis system and placed in the sorbent recharger. The zirconium oxide sorbent module **402** can be fluidly connectable to the zirconium oxide recharging flow path **401** through zirconium oxide sorbent module inlet **403** and zirconium oxide sorbent module outlet **404.** The zirconium oxide recharging flow path **401** can include at least one pump **407** to provide a driving force for moving fluids through the zirconium oxide recharging flow path **401.** In certain embodiments, two or more pumps can be included for individually introducing fluids from the recharge solution sources into the zirconium oxide sorbent module **402.** As described, the system and methods allow for setting a flow rate of the recharge solutions through the zirconium oxide recharging flow path **401** for precision recharging. The zirconium oxide recharging flow path **401** can include one or more recharge solution sources, including a base source **405** fluidly connected to the zirconium oxide recharging flow path **401** through fluid line **409** and optionally a free chlorine source **406** fluidly connected to the zirconium oxide recharging flow path **401** through fluid line **410.** The base source **405** can contain a base solution, such as sodium hydroxide or potassium hydroxide. Depending on the concentration and temperature of the base solution, the base solution can also disinfect the zirconium oxide sorbent module **402.** Alternatively, a free chlorine solution can be used for disinfection. The free chlorine source **406** can contain any free chlorine solution, including a solution of sodium hypochlorite, potassium hypochlorite or trichloroisocyanuric acid. The hydroxide ions in the base solution displace anions, such as phosphate ions, that have been adsorbed by the zirconium oxide during treatment. Although shown as two separate sources in FIG. 4, a single recharge solution source can be used containing both a base solution and a free chlorine solution. If multiple recharge solution sources are used, the recharge solutions can be mixed within the zirconium oxide recharging flow path **401** or pumped through the zirconium oxide sorbent module **402** sequentially. Water from a water source **414** can be used to dilute the base or free chlorine solution if concentrated solutions are used, and to rinse the zirconium oxide sorbent module **402** before and after introducing the base and free chlorine solutions through the zirconium oxide sorbent module **402** through fluid line **415.** Optional valve **408** can be included to control the movement of fluid from r the base source **405**, free chlorine source **406**, or water source **414.** Alternatively, separate pumps on fluid lines fluidly connected to each recharge solution source can be used. A processor (not shown) can be programmed to control the pumps or valves to direct recharge solutions from the recharge solution sources through the zirconium oxide sorbent module **402.** One of skill in the art will understand that multiple pump and valve arrangements can be used to introduce the necessary recharge solutions through the zirconium oxide sorbent module **402.**

A sensor **413** positioned in, or in fluid connection with the effluent line **411** can determine at least one fluid parameter of the effluent recharge solution in effluent line **411.** In certain embodiments, the sensor **413** can be an ion selective electrode, a pH sensor, a conductivity sensor, an ion selective electrode, an optical sensor, or a combination thereof. As described, the sensor **413** in, or in fluid connection with effluent line **411** can be used by the processor to set one or more recharge parameters.

During a dialysis session, the zirconium oxide serves to remove anions from spent dialysate, including phosphate. The hydroxide ions in the base solution displace anions, such as phosphate ions, that have been adsorbed by the zirconium oxide during treatment. The free chlorine acts to disinfect the zirconium oxide sorbent module **402.**

The conductivity or pH of the effluent of the zirconium oxide sorbent module **402** can be used to set one or more recharge parameters, such as the volume of the recharge solutions necessary for recharging the zirconium oxide, the concentration of the recharge solutions, or the flow rate of the recharge solutions. In certain embodiments, the conductivity or pH of the effluent at a given point in the recharging process can be compared to a known elution profile. Using a lookup table, the processor can compare the conductivity or pH to a known elution profile, which can be characterized as described for a zirconium phosphate elution profile, and determine the remaining capacity of the zirconium oxide. The remaining capacity is proportional to the amount of anions adsorbed by the zirconium oxide during dialysis. Based on the remaining capacity of the zirconium oxide in zirconium oxide sorbent module **402**, the processor can determine the volume of recharge solution necessary to restore the zirconium oxide sorbent module **402** to full or nearly full capacity based on a concentration of the recharge solution and/or a flow rate of the recharge solution. Alternatively, the processor can set the flow rate or concentration of the recharge solutions based on data from the sensor using a set volume of recharge solution to be used. The processor can receive the conductivity or pH of the effluent from the zirconium oxide sorbent module **402** at the beginning of the recharging process, after the recharging process has started, or at one or more times during recharging. As with zirconium phosphate recharging, there are interdependencies between concentration, time, volume and flow rate used in recharging zirconium oxide. For example, at a faster flow rate or at a higher recharge solution concentration, recharging the sorbent material may be quicker, but may require additional chemicals. At lower flow rates or recharge solution concentrations the process may be more efficient with respect to chemical usage, but may take longer. A user can input the values that are desired to be minimized based on the user objectives, and the system can determine the proper concentration, flow rate, and/or volume of the recharge solutions to minimize the desired parameters. The system can be adaptive to the needs of the user, with the user defining the desired objectives for the recharging process, such as to minimize time or to minimize chemical usage. In certain embodiments, the recharger can use a higher concentration or flow rate at the beginning of recharging, and adjust the concentration and/or flow rate lower as the recharging process nears completion, as determined by the sensors, minimizing both the time and volume of recharge solutions necessary for recharging the zirconium oxide.

In certain embodiments, the sensor **413** can be an ion selective electrode. An ion selective electrode or optical sensor can be used to determine the concentration of a specific ion in a fluid, such as phosphate anions. A phosphate selective electrode can use a phosphate selective membrane, such as variable mixtures of silver phosphate, silver sulfide, and PTFE or silver phosphate, silver sulfide and nanotube, which have been demonstrated as successful. As recharging progresses, the concentration of the phosphate ions in the effluent will decrease. When the concentration of the phosphate ions in the effluent approaches zero, the zirconium oxide is at or near full capacity and the recharging process is ended.

In certain embodiments, the sensor **413** can be an optical sensor. During recharging, protein fragments may be eluted from the zirconium oxide sorbent module **402.** The protein fragments can be detected using an optical sensor. For example, the absorbance of light at 230 nm can be used to detect the presence and concentration of proteins or protein fragments. As the recharging process nears competition, protein fragments will no longer be eluted from the zirconium oxide sorbent module **402.** As the absorbance of light at 230 nm approaches a background threshold, the processor can stop introduction of the recharge solutions. Optical sensors for measuring phosphate or similar anions are also known in the art. The optical sensor can detect the phosphate anions in the effluent. As the recharging process nears completion, the phosphate concentration in the effluent will approach 0. In certain embodiments, the optical sensor can be a colorimetric sensor. For example, one or more phosphate sensing membranes can be contacted with the effluent, the phosphate sensing membranes changing color or any other optical parameter in response to the phosphate content in the effluent line **411.** The optical change in the phosphate sensing membrane can be detected by a photodetector to determine the phosphate content of the effluent. As the recharging process nears competition, the ammonia and/or ammonium concentration in the effluent will approach 0.

In certain embodiments, a second sensor **412** can be included in, or in fluid connection with the zirconium oxide recharging flow path **401** at a position upstream of the zirconium oxide sorbent module **402.** As the recharging process nears completion, the difference in any fluid parameters of the initial recharge solution and the effluent of the zirconium oxide sorbent module **402** decreases because the zirconium oxide approaches equilibrium with the recharge solutions. As such, the difference in conductivity or pH as measured by the first sensor **413** and second sensor **412** can be used to determine when recharging is complete. In certain embodiments, the processor can be programmed to stop the introduction of recharging fluids when the conductivity or pH of the effluent is within a predetermined range of the conductivity or pH of the initial recharge solutions. As described, the predetermined range can be set based on a desired final recharge state. For example, the predetermined range can be set at or near 0 if full recharging is required or desired. Alternatively, the predetermined range can be set at some non-zero value if a full restoration of capacity is not needed or wanted. Alternatively, the predetermined range can be set at some non-zero value because the void volume of the sorbent cartridge will allow additional recharge solution to go through the sorbent cartridge during the next step of the recharge process. For example, the processor can determine that the next step in the recharge process, such as water rinsing, can be started when a specified non-zero value or concentration is measured at the outlet of the sorbent cartridge because the void volume of the sorbent cartridge will still contain recharge solution that is yet to come out and contact the sensor. As the recharge solution continues to exit the sorbent cartridge during the rinse step, the concentration or measured value will continue to decrease to the predetermined value targeted to indicate the end of the recharge process. One of skill in the art will understand that, if the recharge solutions have a known conductivity or pH, the second sensor **412** is unnecessary.

In certain embodiments, after introducing base solution through the zirconium oxide sorbent module **402,** the zirconium oxide sorbent module **402** can be rinsed with water to remove any base or free chlorine solution remaining. The amount of water used during the final rinse can be set by the processor using the sensor **413**, such as a conductivity or pH sensor. The conductivity or pH of the effluent is a function of the ions and/or base present in solution. As the rinsing nears completion, the conductivity and pH of the effluent will approach that of pure water because the base and free chlorine solutions are washed out of the zirconium oxide sorbent module **402.** The processor can introduce water through the zirconium oxide sorbent module **402** until the conductivity reaches a predetermined range, such as 2-mS/cm or less, and/or until the pH of the effluent approaches 7.

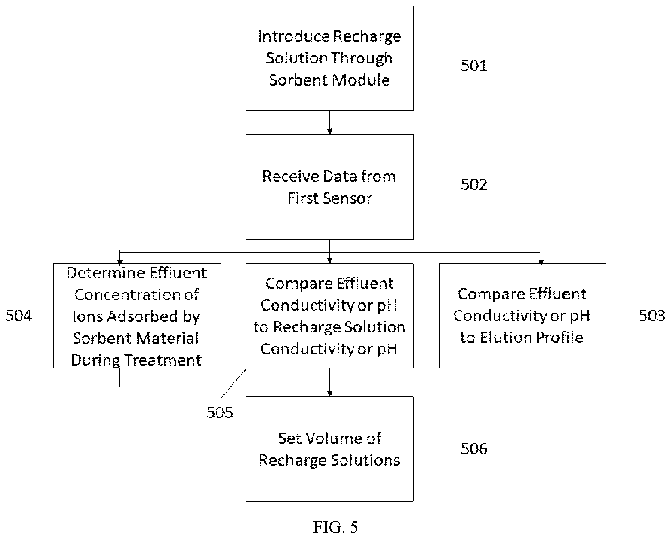

FIG. 5 is a flow chart for performing precision recharging based on a sorbent module effluent analysis during recharging. The sorbent module used in the method of FIG. 5 can contain either zirconium phosphate or zirconium oxide. In step **501**, a recharge solution can be introduced through a sorbent module fluidly connected to a sorbent recharger, as illustrated in FIG.'s 3-4. In step **502** a processor of the sorbent recharger can receive a fluid parameter, such as conductivity, pH, or an ion concentration in an effluent of the sorbent module.

Based on the data received from the sensor in step **502**, the processor can perform any one or more of steps **503-505.** In step **503,** the effluent pH or effluent conductivity from the sorbent module can be compared to an elution profile to determine the remaining capacity of the sorbent material. In step **506**, the processor can set the volume of recharge solution necessary to recharge the sorbent material using a lookup table or algorithm based on the remaining capacity of the sorbent material. In step **504**, the processor can determine an effluent ion concentration. The ion can be ammonium ions, potassium ions, calcium ions, or magnesium ions if the sorbent module contains zirconium phosphate. The ion can be phosphate ions if the sorbent module contains zirconium oxide. In step **506**, the processor can set the volume of recharge solution necessary to recharge the sorbent material, such as by stopping the introduction of the recharge solution when the effluent ion concentration approaches zero. In step **505**, the effluent pH or effluent conductivity from the sorbent module can be compared to a conductivity or pH of the recharge solution. The conductivity or pH of the recharge solution can be determined using a sensor in the recharging flow paths or by using a recharge solution having a known pH or conductivity. In step **506**, the processor can set the volume of recharge solution necessary to recharge the sorbent material, such as by stopping the introduction of the recharge solution when the conductivity or pH of the effluent is within a predetermined range of the conductivity or pH of the recharge solution.

In certain embodiments, two or more of steps **503, 504,** and **505** can be used. For example, effluent parameters, such as effluent pH, effluent conductivity, or effluent ion concentration, can be measured at a beginning of the recharge process. For example, initial values can be compared to an elution profile, shown as step **503.** Based on the comparison of the initial effluent values to the elution profile, recharge solution parameters, such as a volume of recharge solution, a concentration of a recharge solution, or a flow rate of a recharge solution can be set. As the effluent parameters approach the target threshold of steps **504** or **505,** the sensors can be used as further input to the process which can then start to slow the flow rates or change concentrations so as to optimize the process to minimize chemical usage or time. Alternatively, the system can continuously or intermittently compare the effluent parameters to the elution profile. As recharging progresses, the location on the curve of the elution profile can be updated based on more recent information from the sensors. That is, the concentration, volume, and/or flow rate of the recharge solution can be initially set based on initial effluent parameters, and then modified during recharging based on the effluent parameters as the sorbent materials are recharged.

In certain embodiments, the systems and methods can be used to minimize one or more values, such as the time required to recharge the sorbent material or the amount of chemicals needed. As described, there are interdependencies between concentration, time, volume and flow rate. For example, at a faster flow rate or at a higher recharge solution concentration, recharging the sorbent material may be quicker, but may require additional chemicals. At lower flow rates or recharge solution concentrations the process may be more efficient with respect to chemical usage, but may take longer. A user can input the values that are desired to be minimized based on the user objectives, and the system can determine the proper concentration and flow rate of the recharge solutions to minimize the desired parameters. The system can be adaptive to the needs of the user, with the user defining the desired objectives for the recharging process, such as to minimize time or to minimize chemical usage.

In certain embodiments, a user can input additional information concerning the previous dialysis session and sorbent cartridge usage. For example, the user can input whether the zirconium phosphate sorbent module or zirconium oxide sorbent module reached saturation, the length of time the sorbent module has been used, and/or the number of recharge cycles already completed on the sorbent module. Any one or more of the factors can be used to establish recharge performance curves vs. sorbent cartridge usage. Based on recharge performance curves vs. sorbent cartridge usage, recharge parameters can be established either experimentally or through recharge history database that is built and tracked over time. The database can then be utilized to determine an adaptive recharge process that takes into consideration such as recharge cycle, cartridge usage, sensor input, etc.

One skilled in the art will understand that various combinations and/or modifications and variations can be made in the described systems and methods depending upon the specific needs for operation. Moreover, features illustrated or described as being part of an aspect of the invention may be used in the aspect of the invention, either alone or in combination, or follow a preferred arrangement of one or more of the described elements.

The invention may be described by reference to the following numbered paragraphs:-
1. A system, comprising: a sorbent recharger (101), the sorbent recharger comprising a recharging flow path (301, 401), the recharging flow path fluidly connectable to a sorbent module (302, 402) containing at least one sorbent material; at least one recharge solution source (305, 306, 405, 406) containing at least one recharge solution for recharging the at least one sorbent material within the sorbent module, the at least one recharge solution source fluidly connectable to a sorbent module inlet; at least one pump (307, 407) in the recharging flow path; and at least a first sensor (310, 413) in an effluent line (309, 411) fluidly connectable to a sorbent module outlet (304, 404) of the sorbent module; and
   a processor; the processor programmed to receive data from the first sensor and to set at least one recharge parameter for recharging of the at least one sorbent material.
2. The system of paragraph 1, wherein the at least one sorbent material comprises zirconium phosphate.
3. The system of paragraph 1, wherein the at least one sorbent material comprises zirconium oxide.
4. The system of any of paragraphs 1-3, wherein the at least one recharge parameter is selected from the group consisting of a volume of at least one recharge solution introduced through the sorbent module, a concentration of at least one recharge solution introduced through the sorbent module, and a flow rate of at least one recharge solution introduced through the sorbent module.
5. The system of any of paragraphs 1-4, further comprising at least a second sensor (311, 412) positioned between the at least one recharge solution source and the sorbent module inlet.
6. The system of paragraph 5, wherein the processor is programmed to set the at least one recharge parameter based on a difference between data received from the first sensor and data received from the second sensor.
7. The system of any of paragraphs 5-6, wherein the processor is programmed to introduce the at least one recharge solution into the sorbent module until a pH or conductivity in an effluent of the sorbent module is within a predetermined range of a pH or conductivity of the at least one recharge solution.
8. The system of any of paragraphs 1-6, wherein the first sensor is a pH sensor or a conductivity sensor.
9. The system of any of paragraphs 1-6, wherein the first sensor is an ion selective electrode.
10. The system of any of paragraphs 1-6, wherein the first sensor is an optical sensor.
11. The system of any of paragraphs 1-8, wherein the processor is programmed to compare a conductivity or pH in an effluent of the sorbent module to an elution profile.
12. The system of any of paragraphs 1-2, or 5-11, wherein the sorbent module contains zirconium phosphate, and wherein the at least one recharge solution source comprises at least a water source and a brine source.
13. The system of any of paragraphs 1 or 3-11, wherein the sorbent module contains zirconium oxide, and wherein the at least one recharge solution source comprises at least a base source.
14. The system of any of paragraphs 1-13, the system performing precision recharging of the at least one sorbent material.
15. A method, comprising the steps of:
   introducing a recharge solution into a sorbent module (302, 402) containing at least one sorbent material in a sorbent recharger (101);
   receiving data from a first sensor (310, 413) positioned in an effluent line (309, 411) fluidly connected to a sorbent module outlet (304, 404) of the sorbent module; and
   setting at least one recharge parameter based on data received from the first sensor.
16. The method of paragraph 15, wherein the sorbent module contains zirconium phosphate.
17. The method of paragraph 15, wherein the sorbent module contains zirconium oxide.
18. The method of any of paragraphs 15-17, wherein the at least one recharge parameter is selected from the group consisting of a volume of at least one recharge solution introduced through the sorbent module, a concentration of at least one recharge solution introduced through the sorbent module, and a flow rate of at least one recharge solution introduced through the sorbent module.
19. The method of any of paragraphs 15-17, further comprising the step of receiving data from a second sensor (311, 412) positioned between a recharge solution source containing the recharge solution and a sorbent module inlet of the sorbent module and comparing the data from the second sensor to data from the first sensor.
20. The method of paragraph 19, further comprising the step of stopping introduction of the recharge solution when data received from the first sensor is within a predetermined range of data received from the second sensor.
21. The method of any of paragraphs 15-20, wherein the first sensor is a pH sensor, a conductivity sensor, an ion selective electrode, or an optical sensor.
22. The method of paragraph 16, wherein the recharge solution comprises at least a brine solution.
23. The method of paragraph 17, wherein the recharge solution comprises at least a base solution.
24. The method of paragraph 15, wherein the step of setting the at least one recharge parameter comprises comparing data from the first sensor to an elution profile of the sorbent module.
25. The method of paragraph 21, wherein the first sensor is an ion selective electrode, and further comprising the step of stopping introduction of the recharge solution when a concentration of at least one ion in the effluent line is within a predetermined range.
26. The method of paragraph 25, wherein the predetermined range is a non-zero value.
27. The method of any of paragraphs 15-26, wherein the method is performed by the system of paragraph 1.
28. The method of any of paragraphs 15-27, wherein the at least one recharge parameter is set based on data received from the first sensor at a first time, and wherein the at least one recharge parameter is adjusted based on data received from the first sensor at a second time.
29. The method of any of paragraphs 15-28, the method for precision recharging of the at least one sorbent material.

## Claims

1. A system, comprising:
a sorbent recharger (101), the sorbent recharger comprising a recharging flow path (301, 401), the recharging flow path fluidly connectable to a sorbent module (302, 402) containing at least one sorbent material; at least one recharge solution source (305, 306, 405, 406) containing at least one recharge solution for recharging the at least one sorbent material within the sorbent module, the at least one recharge solution source fluidly connectable to a sorbent module inlet; at least one pump (307, 407) in the recharging flow path; and at least a first sensor (310, 413) in an effluent line (309, 411) fluidly connectable to a sorbent module outlet (304, 404) of the sorbent module; and
a processor; the processor programmed to receive data from the first sensor and to set at least one recharge parameter for recharging of the at least one sorbent material.

2. The system of claim 1, wherein the at least one sorbent material comprises zirconium phosphate, or wherein the at least one sorbent material comprises zirconium oxide.

3. The system of any of claims 1-2, wherein the at least one recharge parameter is selected from the group consisting of a volume of at least one recharge solution introduced through the sorbent module, a concentration of at least one recharge solution introduced through the sorbent module, and a flow rate of at least one recharge solution introduced through the sorbent module.

4. The system of any of claims 1-3, further comprising at least a second sensor (311, 412) positioned between the at least one recharge solution source and the sorbent module inlet.

5. The system of claim 4, wherein the processor is programmed to set the at least one recharge parameter based on a difference between data received from the first sensor and data received from the second sensor.

6. The system of any of claims 4-5, wherein the processor is programmed to introduce the at least one recharge solution into the sorbent module until a pH or conductivity in an effluent of the sorbent module is within a predetermined range of a pH or conductivity of the at least one recharge solution.

7. The system of any of claims 1-6, wherein the first sensor is a pH sensor,conductivity sensor,
an ion selective electrode, or an optical sensor.

8. The system of any of claims 1-7, wherein the processor is programmed to compare a conductivity or pH in an effluent of the sorbent module to an elution profile.

9. The system of any of claims 1-8, the system performing precision recharging of the at least one sorbent material.

10. A method, comprising the steps of:
introducing a recharge solution into a sorbent module (302, 402) containing at least one sorbent material in a sorbent recharger (101);
receiving data from a first sensor (310, 413) positioned in an effluent line (309, 411) fluidly connected to a sorbent module outlet (304, 404) of the sorbent module; and
setting at least one recharge parameter based on data received from the first sensor.

11. The method of claim 10, wherein the sorbent module contains zirconium phosphate, or wherein the sorbent module contains zirconium oxide.

12. The method of any of claims 10-11, wherein the at least one recharge parameter is selected from the group consisting of a volume of at least one recharge solution introduced through the sorbent module, a concentration of at least one recharge solution introduced through the sorbent module, and a flow rate of at least one recharge solution introduced through the sorbent module.

13. The method of any of claims 10-12, further comprising the step of receiving data from a second sensor (311, 412) positioned between a recharge solution source containing the recharge solution and a sorbent module inlet of the sorbent module and comparing the data from the second sensor to data from the first sensor.

14. The method of claim 13, further comprising the step of stopping introduction of the recharge solution when data received from the first sensor is within a predetermined range of data received from the second sensor.

15. The method of any of claims 10-14, wherein the first sensor is a pH sensor, a conductivity sensor, an ion selective electrode, or an optical sensor.
